# EUROPÄISCHE PATENTSCHRIFT

(11) **EP 0 584 526 B1**
(45) Veröffentlichungstag und Bekanntmachung des Hinweises auf die Patenterteilung: **26.03.1997**
(21) Anmeldenummer: 93111548.9
(22) Anmeldetag: 19.07.1993
(51) Int. Cl.: A61N 1/05

(54) **Steuerbare Elektrodenvorrichtung**
Controllable electrode device
Dispositif d'électrode commandé

(30) Priorität: 28.08.1992 SE 9202481
(43) Veröffentlichungstag der Anmeldung: 02.03.1994
(73) Patentinhaber: Pacesetter AB, 171 95 Solna (SE)
(72) Erfinder: Nyman, Per, S-182 64 Djursholm (SE)
(74) Vertreter: Lettström, Richard Wilhelm

(56) Entgegenhaltungen:
- US-A- 4 498 482
- US-A- 4 677 990
- US-A- 4 791 939

## Beschreibung

Steuerbare Elektrodenvorrichtung zur intrakorporalen Stimulation von Körpergeweben, insbesondere zur intrakardialen Stimulation des Herzens mit einem Elektrodenkabel, das einen langgestreckten, flexiblen Leiter umfasst, dessen Aussenseite mit einer Isolierschicht versehen ist und dessen Innenseite einen Kanal zur Einführung eines langgestreckten Organes zur Übertragung einer Kraft in Richtung zum distalen Ende des Elektrodenkabels bildet und mit einem Elektrodenkopf, der am distalen Ende des Leiters angebracht ist.

Es ist von grosser Bedeutung, dass das Elektrodenkabel bei einer derartigen Elektrodenvorrichtung ausreichend weich ist, damit es bei einer Einführung in das Herz eines Patienten über eine Vene dieser folgen kann, ohne die Venenwände zu beschädigen. Die Einführung des Elektrodenkabels erfolgt mit Hilfe eines Mandrins, der in den Kanal des Elektrodenkabels eingeführt wird. Der Mandrin besteht aus einem Material, das ausreichend steif ist, damit das Elektrodenkabel in einer Vene verschoben werden kann. In schwierigen Passagen, in denen das Elektrodenkabel stark gebogen werden muss, wird der Mandrin häufig etwas nach hinten verschoben, so dass das distale Ende des Elektrodenkabels maximal biegsam wird. Nachdem das vordere Ende des Elektrodenkabels eine solche Stelle passiert hat, wird der Mandrin wieder an das distale Ende vorgeschoben, damit dieses Ende zum Vorhof oder zur Kammer des Herzens transportiert werden kann, bis der Elektrodenkopf gegen die Herzwand zur Stimulation anliegt. Wenn das Elektrodenkabel am Platz ist, wird der Mandrin aus dem Kabel herausgezogen. Bei scharfen Venenkrümmungen kann der Mandrin unter Umständen den Leiter, der häufig den Kanal bildet, beschädigen.

Eine Elektrodenvorrichtung der eingangs genannten Art, deren transportierbar ist, ist in der DE-OS 3 043 189 beschrieben.

Eine weitere Elektrodenvorrichtung dieser Art ist aus der US-PS 4 677 990 bekannt. Der hier beschriebene Mandrin ist im Vergleich zum übrigen Mandrin an seinem vorderen Ende dünner, damit das distale Ende des Elektrodenkabels in eine J-Form gebogen werden kann.

Der Erfindung liegt die Aufgabe zugrunde, eine Elektrodenvorrichtung der eingangs genannten Art mit einem Elektrodenkabel zu schaffen, das auch mit einem eingeführten Mandrin verhältnismässig biegsam und elastisch ist. Der Mandrin muss auch nicht notwendigerweise während eines Transportes des Elektrodenkabels durch eine Vene in dem Elektrodenkabel verschoben werden.

Diese Aufgabe ist erfindungsgemäss dadurch gelöst, dass das langgestreckte Organ so ausgebildet ist, dass es in der Längsrichtung steif ist und eine verhältnismässig hohe seitliche Flexibilität aufweist. Wenn das langgestreckte Organ bzw. der Mandrin im Kanal des Elektrodenkabels appliziert ist, ist der Mandrin durch den Leiter und die Isolierschicht seitlich gestützt. Dadurch, dass der Mandrin in der Längsrichtung steif ist, kann er die für den Transport notwendige schiebende Kraft an das distale Ende des Elektrodenkabels übertragen. Durch diesen seitlich verhältnismässig geschmeidigen und anpassungsfähigen Mandrin ist es nicht mehr notwendig, dass er während des beschriebenen Transportes in einer Vene im Verhältnis zum Elektrodenkabel verschoben werden muss.

Gemäß der Erfindung besteht das langgestreckte Organ aus mindestens zwei Drähten, die verdrillt sind. Hierdurch kann eine sehr hohe seitliche Geschmeidigkeit erhalten werden gleichzeitig damit, dass eine verhältnismässig hohe Steifigkeit in der Längsrichtung gegeben ist.

Nach Weiterbildungen der Erfindung können die verdrillten Drähte vorzugsweise aus Metall oder aus einem Kunststoffmaterial sein.

Die Erfindung ist nachfolgend anhand von einem in der Zeichnung dargestellten Ausführungsbeispiel näher erläutert. Es zeigt:

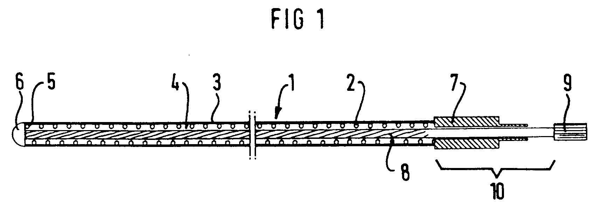

- FIG 1: eine Seitenansicht einer Elektrodenvorrichtung in einem Längsschnitt mit einem Mandrin nach der Erfindung.

In der FIG 1 ist eine Elektrodenvorrichtung zur intrakardialen Stimulation eines Herzens abgebildet. Die Elektrodenvorrichtung weist ein Elektrodenkabel 1 auf, das einen langgestreckten, flexiblen Leiter 2 umfasst, dessen Aussenseite mit einer Isolierschicht 3 versehen ist und dessen Innenseite einen Kanal 4 bildet. Am distalen Ende 5 des Leiters 2 ist ein Elektrodenkopf 6 zur Stimulation des Herzgewebes eines Patienten angebracht. Das Elektrodenkabel 1 umfasst auch ein Anschlussteil 7, das zum Anschliessen des Kabels 1 an ein in dieser FIG nicht dargestelltes Herzstimulationsgerät vorgesehen ist. In den Kanal 4 ist ein Mandrin 8, der aus mindestens zwei verdrillten Drähten besteht, eingeführt. Die Drähte können aus Metall oder aus einem Kunststoffmaterial bestehen. Das proximale Ende des Mandrins 8 ist mit einem Griff 9 versehen. Eine Strecke 10 des Mandrins 8, die im Bereich des Griffes 9 liegt, ist in diesem Ausführungsbeispiel z.B. durch Verzinnen seitlich versteift, wenn der Mandrin 8 aus Metall ist oder z.B. durch Leim, wenn der Mandrin 8 aus einem Kunststoffmaterial ist. Eine solche Versteifung kann insbesondere dann von Vorteil sein, wenn dieses Teil des Mandrins 8 ausserhalb des Elektrodenkabels 1 liegt und nicht mit Hilfe des Leiters 2 und der Isolierschicht 3 seitlich versteift wird. Da das Elektrodenkabel 1 insbesondere im distalen Bereich Endes und im mittleren Bereich Geschmeidigkeit fordert, können auch andere Teile bzw. Strecken des Mandrins 8 in beschriebener Weise versteift werden.

Die Erfindung ist nicht auf die hier gezeigten und beschriebenen Ausführungsbeispiele beschränkt. Das Ziel der Erfindung ist es, einen in der Längsrichtung mit der vorhandenen seitlichen Stütze des Elektrodenkabels steif vorschiebbaren Mandrin mit einer hohen seitlichen Flexibilität zu erhalten. Der Vorteil mit der vorgeschlagenen Erfindung besteht in erster Linie darin eine im Vergleich zu bekannten Elektrodenvorrichtungen mit Mandrinen erleichterte Einführung eines Elektrodenkabels in eine Vene zu erhalten. Dadurch, dass das Elektrodenkabel mit fast beibehaltener Biegsamkeit in eine Vene eingeführt werden kann, werden Risiken in Bezug auf unerwünschte Venenspasmen, Schäden an Venen sowie verkehrtes Einführen in die Koronargefässe und in die unteren Venen wie z.B. in vena cava inferior verringert. Durch den seitlich geschmeidigen Mandrin wird auch das Risiko für Schäden am Leiter des Elektrodenkabels beim Herausziehen des Mandrins aus dem Kanal des Elektrodenkabels verringert.

### Bezugszeichenliste

- 1: Elektrodenkabel
- 2: Leiter
- 3: Isolierschicht
- 4: Kanal
- 5: das distale Ende des Leiters
- 6: Elektrodenkopf
- 7: Anschlussteil
- 8,: Mandrin, langgestrecktes Organ, Draht
- 9: Griff
- 10,: Strecke

## Patentansprüche

1. Steuerbare Elektrodenvorrichtung zur intrakorporalen Stimulation von Körpergeweben, insbesondere zur intrakardialen Stimulation des Herzens, mit einem Elektrodenkabel (1), das einen langgestreckten, flexiblen Leiter (2) umfasst, dessen Aussenseite mit einer Isolierschicht (3) versehen ist und dessen Innenseite einen Kanal (4) bildet, und das ein distales Ende mit einem Elektrodenkopf (6) und ein proximales Ende aufweist, und mit einem langgestreckten Organ (8, 11) im Kanal (4) zur Übertragung einer Kraft an das distale Ende des Elektrodenkabels, wobei das langgestreckte Organ (8, 11) so ausgebildet ist, dass es in der Längsrichtung steif ist und eine verhältnismässig hohe seitliche Flexibilität aufweist, **dadurch gekennzeichnet**, dass das langgestreckte Organ (8, 11) aus mindestens zwei Drähten besteht, die verdrillt sind.

2. Steuerbare Elektrodenvorrichtung nach Anspruch 1, **dadurch gekennzeichnet**, dass die Drähte (8) aus einem Kunstoffmaterial sind.

3. Steuerbare Elektrodenvorrichtung nach Anspruch 1, **dadurch gekennzeichnet**, dass die Drähte (8) aus Metall sind.

## Claims

1. Controllable electrode device for intracorporeal stimulation of body tissues, in particular for intracardiac stimulation of the heart, having an electrode cable (1), which comprises an elongated, flexible conductor (2), the outside of which is provided with an insulating layer (3) and the inside of which forms a channel (4), and which has a distal end having an electrode head (6) and a proximal end, and having an elongated element (8, 11) in the channel (4) for transmitting a force to the distal end of the electrode cable, with the elongated element (8, 11) being formed in such a way that it is rigid in the longitudinal direction and has a comparatively high lateral flexibility, characterised in that the elongated element (8, 11) consists of at least two wires, which are twisted.

2. Controllable electrode device according to claim 1, characterised in that the wires (8) consist of a plastics material.

3. Controllable electrode according to claim 1, characterised in that the wires (8) consist of metal.

## Revendications

1. Dispositif d'électrodes pouvant être commandé pour stimuler intracorporellement des tissus de corps, notamment pour stimuler de manière intracardiaque le coeur, comportant un câble (1) d'électrode, qui comprend un conducteur (2) souple oblong, dont le côté extérieur est muni d'une couche (3) isolante et dont le côté intérieur forme un canal (4), et qui comprend une extrémité distale munie d'une tête (6) d'électrode et une extrémité proximale, et comportant un organe (8,11) oblong dans le canal (4) pour transmettre une force à l'extrémité distale du câble d'électrode, l'organe (8,11) oblong étant formé de manière qu'il soit rigide suivant la direction longitudinale et ait une souplesse latérale proportionnelle à la hauteur, caractérisé en ce que l'organe (8,11) oblong est constitué d'au moins deux fils, qui sont torsadés.

2. Dispositif d'électrodes suivant la revendication 1, caractérisé en ce que les fils sont en une matière plastique.

3. Dispositif d'électrodes suivant la revendication 1, caractérisé en ce que les fils (8) sont en métal.
